# EUROPEAN PATENT APPLICATION

(11) **EP 4 814 005 A1**
(43) Date of publication of application: **30.09.2026**
(21) Application number: 24894360.7
(22) Date of filing: 30.09.2024
(51) Int. Cl.: C07K 14/705, C07K 14/725, C07K 14/36, C07K 16/30, C12N 5/0783, A61K 39/00, A61P 35/00

(54) **ANTICANCER COMPOSITION COMPRISING CHIMERIC ANTIGEN RECEPTOR-EXPRESSING IMMUNE CELL AND TRAPTAVIDIN SCAFFOLD ANTIBODY-LIKE PROTEIN, AND USE THEREOF**

(30) Priority: 20.11.2023 KR 20230161607
(71) Applicant: Scripps Korea Antibody Institute, Chuncheon-si, Gangwon-do 24341 (KR); Korea Institute of Science and Technology, Seoul 02792 (KR)
(72) Inventor: LEE, Sungjin, Chuncheon-si Gangwon-do 24363 (KR); KIM, Youngyoub, Incheon 21443 (KR); BANG, Youngbin, Gyeonggi-do 16516 (KR); KO, Hae Li, Chuncheon-si Gangwon-do 24435 (KR); LEE, Deuk Ki, Chuncheon-si Gangwon-do 24434 (KR); KIM, Younghyun, Namyangju-si Gyeonggi-do 12219 (KR); JUNG, Sang Hoon, Seoul 05502 (KR); LEE, Jae Wook, Gangneung-si Gangwon-do 25522 (KR)
(74) Representative: Longland, Emma Louise
(86) International application number: PCT/KR2024/014872
(87) International publication number: WO 2025/110464

(57) **Abstract**

The present invention relates to an anticancer cell therapeutic composition comprising immune cells expressing chimeric antigen receptors and traptavidin scaffold antibody-like proteins and a use thereof. The invention provides: an immune cell expressing a chimeric antigen receptor (CAR) that includes a biotin-tagged protein, and a co-stimulatory domain and an activation domain which are both capable of inducing an anticancer response; an antibody-like protein that contains a single-chain variable fragment (scFv) binding specifically to a tumor antigen, and traptavidin; wherein the antibody-like protein forms a tetramer through the self-binding of traptavidin and binds to a biotin-tagged protein expressed on the surface of the immune cell to allow the immune cell to have four single-chain variable fragments (scFvs), thereby providing an immune cell therapeutic agent having enhanced avidity to a tumor-specific antigen.

## Description

### CROSS-REFERENCE TO RELATED APPLICATIONS

The present invention relates to an anti-cancer cell therapeutic composition comprising an immune cell expressing a chimeric antigen receptor and a Traptavidin scaffold antibody like protein, and use thereof.

### BACKGROUND

When normal cells are genetically or acquiredly modified to exhibit abnormal proliferation, the normal cells are converted into cancer cells, and cancer develops due to proliferation of the cancer cells. Cancer cells express proteins and genes that are differentiated from normal cells, and these are referred to as tumor antigens capable of distinguishing cancer cells. Tumor antigens are recognized by NK cells, T cells, and B cells, which are immune systems inherently possessed by the body, and are utilized as means for killing cancer, and are utilized as target substances of various anti-cancer agents. However, in specific cancers, development of an anti-cancer agent having improved targeting ability to cancer is required by avoiding various mechanisms by which the immune system of the body and anti-cancer agents target cancer cells.

Anti-cancer agents currently being developed include cell therapeutics using the immune system, and the cell therapeutics are modified into a form in which targeting ability to cancer is improved or anti-cancer activity is enhanced based on immune cells. In order to modify the function of immune cells used as cell therapeutics, a chimeric antigen receptor (CAR) is mainly used, and the currently developed chimeric antigen receptor includes an antigen-binding domain, a transmembrane domain, and one or more intracellular signal transduction domains. In the chimeric antigen receptor, the intracellular signal transduction domain is associated with activation of immune cells, and may initiate, for example, release of cytolytic molecules that induce killing of tumor cells.

Most cell therapeutics currently used utilize T cells. T cells used for cell therapeutics are easy to proliferate and modify in phenotype, and have excellent killing ability against cancer cells. A cell therapeutic using T cells may be modified so that the T cells can be targeted to a specific cancer, and may be engineered to have a tumor recognition site that specifically binds to a specific tumor. As a method for modifying the T cells, a chimeric antigen receptor (CAR) may be used, and T cells expressing the chimeric antigen receptor (CAR) are named CAR-T.

### [Prior Art Documents]

### [Patent Documents]

Korean Patent Application Publication No. 10-2018-0130534 (published on Dec. 07, 2018)

### SUMMARY OF THE INVENTION

### Problem to be Solved by the Invention

An object of the present invention is to provide an immune cell therapeutic having excellent anti-cancer effect due to enhanced binding ability (avidity) to a tumor-specific antigen. In the present invention, an immune cell expressing a chimeric antigen receptor comprising a biotin tag protein and a costimulatory region and a stimulatory region for inducing an anti-cancer response is provided, and an antibody-like protein comprising a single chain variable fragment (scFv) specifically binding to a tumor-specific antigen and Traptavidin is provided, wherein the antibody-like protein forms a tetramer due to self-assembly of Traptavidin, and binds to the biotin tag protein expressed on the surface of the immune cell so that the immune cell is configured to have four single chain variable fragments (scFv), thereby providing an immune cell therapeutic having enhanced binding ability (avidity) to a tumor-specific antigen.

### Means for solving the Problem

The present invention provides a chimeric antigen receptor comprising a tag protein, an HTM region (hinge and transmembrane region), a costimulatory region, and a stimulatory region.

In addition, the present invention provides a polynucleotide encoding the chimeric antigen receptor.

In addition, the present invention provides a vector for manufacturing the chimeric antigen receptor comprising the polynucleotide.

In addition, the present invention provides an immune cell comprising a chimeric antigen receptor comprising a tag protein, an HTM region (hinge and transmembrane region), a costimulatory region, and a stimulatory region, and expressing the tag protein on a cell membrane surface.

In addition, the present invention provides a method for manufacturing a CAR-expressing immune cell, the method comprising transducing an immune cell with a vector comprising a polynucleotide encoding a chimeric antigen receptor comprising a tag protein, an HTM region (hinge and transmembrane region), a costimulatory region, and a stimulatory region.

In addition, the present invention provides an antibody-like protein comprising a single chain variable fragment (scFv) and Traptavidin.

In addition, the present invention provides a polynucleotide encoding the antibody-like protein.

In addition, the present invention provides a vector for manufacturing the antibody-like protein comprising the polynucleotide.

In addition, the present invention provides an immune cell therapeutic composition for preventing or treating cancer, the composition comprising: an immune cell comprising a chimeric antigen receptor comprising a tag protein, an HTM region (hinge and transmembrane region), a costimulatory region, and a stimulatory region, and expressing the tag protein on a cell membrane surface; and an antibody-like protein comprising a single chain variable fragment (scFv) and Traptavidin.

### Effects of the Invention

According to the present invention, an immune cell expressing a chimeric antigen receptor comprising a costimulatory region and a stimulatory region for inducing an anti-cancer response inside the cell while expressing a biotin tag on a membrane surface is provided, and an antibody-like protein composed of a single chain variable fragment (scFv) and Traptavidin binds, in the form of a tetramer due to self-assembly, to the biotin tag expressed on the surface of the immune cell. Therefore, a cell therapeutic manufactured according to the present invention has four single chain variable fragments (scFv) on a cell membrane surface, and thus has enhanced binding ability (avidity) to a tumor-specific antigen, and may be provided as an anti-cancer cell therapeutic having excellent anti-cancer effect.

### BRIEF DESCRIPTION OF THE DRAWINGS

FIG. 1 is a schematic diagram of a plasmid for manufacturing a chimeric antigen receptor according to the present invention.
FIG. 2 shows results obtained by analyzing, by flow cytometry, an expression level of GFP (green fluorescent protein) in order to confirm whether CD4⁺/CD8⁺ T cells expressing a chimeric antigen receptor (CAR) manufactured according to the present invention were produced.
FIG. 3 is a schematic diagram for producing biotin-tagged CAR-T using biotin ligation from T cells (CAR-T) expressing a chimeric antigen receptor (CAR) manufactured according to the present invention.
FIG. 4 shows results obtained by observing fluorescent portions by confocal microscopy in order to confirm whether a biotin protein is exposed on the surface of T cells (CAR-T) transduced with a biotin-tagged CAR protein manufactured according to the present invention.
FIG. 5 is a schematic diagram of a vector for manufacturing a Traptavidin scaffold antibody-like protein according to the present invention.
FIG. 6 shows results obtained by detecting a Traptavidin scaffold antibody-like protein manufactured according to the present invention by SDS-PAGE.
FIG. 7 is a schematic diagram showing a tetramer form formed by self-assembly of the Traptavidin scaffold antibody-like protein manufactured according to the present invention.
FIG. 8 shows Western blot results for confirming the presence of a biotin binding site and FMC63 of the Traptavidin scaffold antibody-like protein manufactured according to the present invention.
FIG. 9 is a schematic diagram of an immune cell therapeutic comprising tetramer based universal CAR-T composed of T cells (CAR-T) transduced with a biotin-tagged CAR protein and a Traptavidin scaffold antibody-like protein according to the present invention.
FIG. 10 shows results obtained by evaluating an effect on cytokine levels in order to evaluate anti-cancer activity of tetramer based universal CAR-T manufactured according to the present invention.
FIG. 11 shows results obtained by evaluating cytotoxicity against cancer cells in order to evaluate anti-cancer activity of tetramer based universal CAR-T manufactured according to the present invention.

### DETAILED DESCRIPTION OF THE INVENTION

Terms used in the present specification have been selected from general terms that are currently widely used as much as possible while considering functions in the present invention, but may vary depending on the intention or precedent of a person skilled in the art, emergence of new technology, and the like. In addition, in a specific case, there is also a term arbitrarily selected by the applicant, and in this case, the meaning thereof will be described in detail in the corresponding description part of the invention. Therefore, the terms used in the present invention should be defined based on the meaning of the terms and the overall content of the present invention, rather than merely the names of the terms.

Unless otherwise defined, all terms used herein comprising technical or scientific terms have the same meanings as those generally understood by a person having ordinary skill in the technical field to which the present invention pertains. Terms such as those defined in commonly used dictionaries should be construed as having meanings consistent with meanings in the context of the related art, and should not be construed as idealized or excessively formal meanings unless clearly defined in the present application.

Numerical ranges include numerical values defined in the ranges. All maximum numerical limitations given throughout the present specification include all lower numerical limitations as if the lower numerical limitations were expressly written. All minimum numerical limitations given throughout the present specification include all higher numerical limitations as if the higher numerical limitations were expressly written. All numerical limitations given throughout the present specification will include all better numerical ranges within broader numerical ranges, as if narrower numerical limitations were expressly written.

Hereinafter, the present invention will be described in more detail.

The present invention provides a chimeric antigen receptor comprising a tag protein, an HTM region (hinge and transmembrane region), a costimulatory region, and a stimulatory region.

The tag protein is an Avidin or a Biotin tag protein.

The "HTM region (hinge and transmembrane region)" means a hydrophobic polypeptide that is naturally or artificially designed and spans from a cell membrane exposed to the outside of a cell to a cell membrane exposed to the inside of the cell or cytoplasm, and is also called a transmembrane domain. The HTM region may be in the form of an alpha helix or beta barrel, or a combination thereof, and may include a multipass protein having a plurality of membrane-spanning segments, each alpha-helical, beta sheet, or a combination thereof.

In one embodiment of the present invention, the HTM region (hinge and transmembrane region) included in the chimeric antigen receptor may be CD8 HTM represented by SEQ ID NO: 3, but is not limited thereto, and the HTM region may be selected or modified by amino acid substitution.

The "costimulatory region" is a polypeptide located inside a cell in order to stimulate or activate part or all of a signal peptide that transmits a signal for cellular activity such as an anti-cancer response or an immune response.

In one embodiment of the present invention, the costimulatory region may be one or more proteins selected from the group consisting of 4-1BB, CD2, CD7, CD27, CD28, CD30, CD40, CD83, CD258, NKG2C, NKG2D, B7-H3, OX40, ICAM-1, LFA-1, and ICOS, or active regions thereof, but is not limited thereto. Preferably, the costimulatory region of the present invention may be 4-1BB represented by SEQ ID NO: 5.

The "stimulatory region" is a signal peptide located inside a cell and transmits a signal for cellular activity such as an anti-cancer response or an immune response.

In one embodiment of the present invention, the stimulatory region may be one or more proteins selected from the group consisting of CD3ζ, CD3ε, CD3γ, CD3δ, TCRα, TCRβ, TCRδ, TCRγ, CD79a, CD79b, DAP10, and DAP12, or active regions thereof, but is not limited thereto. Preferably, the stimulatory region of the present invention may be CD3ζ represented by SEQ ID NO: 7.

The "chimeric antigen receptor" is a recombinant receptor protein having an antigen recognition site that specifically binds to a cancer cell, and includes a site recognizing a surface molecule of the cancer cell, a transmembrane region penetrating a cell membrane, and an intracellular signal transduction site inducing cellular activity. In one embodiment of the present invention, the chimeric antigen receptor includes an Avidin or a Biotin tag protein as a tag protein, includes a CD8 HTM region as a transmembrane region, includes 4-1BB and CD3ζ as intracellular signal transduction sites, and may further include GFP (green fluorescent protein) which is a fluorescent protein. Specifically, the chimeric antigen receptor of the present invention is configured to sequentially include a tag protein, CD8 HTM, 4-1BB, and CD3ζ, and may include GFP (green fluorescent protein) which is a fluorescent protein at the terminus of CD3ζ. More specifically, the chimeric antigen receptor of the present invention may be composed of a polypeptide represented by SEQ ID NO: 11.

The term "protein" used in the present invention is used interchangeably with "peptide" and "polypeptide", and refers to a compound having amino acid residues covalently linked by peptide bonds. A protein or peptide should include at least two amino acids, and there is no limitation on the maximum number of amino acids that may include a sequence of the protein or peptide. A polypeptide includes any peptide or protein having two or more amino acids linked to each other by peptide bonds. The term used herein refers to both two chains, and a short chain is also commonly referred to in the art, for example, as a peptide, oligopeptide, and oligomer, and a longer chain is generally referred to in the art as a protein, and many types belong thereto. "Polypeptide" includes, for example, biologically active fragments, substantially homologous polypeptides, oligopeptides, homodimers, heterodimers, variants of polypeptides, modified polypeptides, derivatives, analogs, fusion proteins, and the like. A polypeptide includes a natural peptide, a recombinant peptide, a synthetic peptide, or a combination thereof.

In addition, the present invention provides a polynucleotide encoding the chimeric antigen receptor.

The "polynucleotide" is a chain of nucleotides and includes DNA and RNA. Specifically, the polynucleotide is a nucleic acid and is a polymer of nucleotides, wherein the polynucleotide may be hydrolyzed into monomeric nucleotides. The polynucleotide includes, but is not limited to, all nucleic acid sequences obtained by recombinant means, that is, any means available in the art comprising conventional cloning techniques and cloning of nucleic acid sequences from recombinant libraries or cellular genomes using polymerase chain reaction (PCR) and the like, and synthetic means.

In addition, the present invention provides a vector for manufacturing the chimeric antigen receptor comprising the polynucleotide.

The vector for manufacturing the chimeric antigen receptor includes an Avidin gene encoding an Avidin tag protein represented by SEQ ID NO: 2; a CD8 HTM gene encoding CD8 HTM represented by SEQ ID NO: 4; a 4-1BB gene encoding 4-1BB represented by SEQ ID NO: 6; and a CD3ζ gene encoding CD3ζ represented by SEQ ID NO: 8. In addition, the vector includes a first linker gene represented by the 46th to 51st nucleotide sequences of SEQ ID NO: 12 between the Avidin gene and the CD8 HTM gene. Additionally, the vector for manufacturing the chimeric antigen receptor may include a gene encoding GFP (green fluorescent protein) represented by SEQ ID NO: 10 at the 3' end of the CD3ζ gene, which is a stimulatory region, and may include a second linker gene represented by the 721st to 735th nucleotide sequences of SEQ ID NO: 12 between the CD3ζ gene and the GFP gene.

Specifically, the vector for manufacturing the chimeric antigen receptor of the present invention may include a polynucleotide represented by SEQ ID NO: 12.

In the present invention, the "vector" is a tool enabling stable transfection of a cell, and the vector enables incorporation of transgene(s) into a cell genome. Preferably, such a vector has a positive selection marker, and a suitable positive selection marker includes any gene that allows a cell to grow under conditions that kill a cell not expressing the gene. Non-limiting examples may include antibiotic resistance. Additionally, the vector is a plasmid vector, and more specifically the vector is a viral vector, and may be replaced with and used as any suitable vector by a person skilled in the art. Preferably, the vector may be a lentiviral vector, a retroviral vector, a DNA vector, a plasmid, an RNA vector, an adenoviral vector, or an adeno-associated viral vector, but is not limited thereto.

Hereinafter, repetitive features shared in each aspect of the invention are omitted from description.

In addition, the present invention provides an immune cell comprising a chimeric antigen receptor comprising a tag protein, an HTM region (hinge and transmembrane region), a costimulatory region, and a stimulatory region, and expressing the tag protein on a cell membrane surface.

The immune cell may be a T cell, an NK cell, or a B cell, but is not limited thereto.

In addition, the present invention provides a method for manufacturing a CAR-expressing immune cell, the method comprising transducing an immune cell with a vector comprising a polynucleotide encoding a chimeric antigen receptor comprising a tag protein, an HTM region (hinge and transmembrane region), a costimulatory region, and a stimulatory region.

When the tag protein is an Avidin, the method may further include a biotin ligation step by reacting with biotin AMP (biotin-adenosine mono phosphate) after the transducing step.

In addition, the present invention provides an antibody-like protein comprising a single chain variable fragment (scFv) and Traptavidin.

The single chain variable fragment (scFv) binds to a tumor-specific antigen, and the tumor-specific antigen may be CD19, CD20, GD2, HER-2, CD30, EGFR, FAP, CD33, CD123, PD-L1, IGF1R, CSPG4, or B7-H4, but is not limited thereto. Specifically, the single chain variable fragment (scFv) includes an FMC63 VL region (variable light chain region), which is a polypeptide represented by SEQ ID NO: 13, and an FMC63 VH region (variable heavy chain region), which is a polypeptide represented by SEQ ID NO: 15.

The Traptavidin is a biotin-binding protein having improved affinity, and is a protein in which S52G and R53D mutations occur in Streptavidin. The Traptavidin has a dissociation rate for biotin that is 10 times slower than that of Streptavidin, and has features of increased mechanical strength and improved thermal stability. In addition, the Traptavidin may form a tetramer due to self-assembly without additional stimulation, a monomer of the Traptavidin has a size of 42 kDa, and a tetramer of the Traptavidin has a size of 168 kDa. Specifically, the Traptavidin of the present invention is a polypeptide represented by SEQ ID NO: 17.

In addition, the present invention provides a polynucleotide encoding the antibody-like protein.

In addition, the present invention provides a vector for manufacturing the antibody-like protein comprising the polynucleotide.

The vector for manufacturing the antibody-like protein includes a leader sequence represented by SEQ ID NO: 22; a gene encoding the FMC63 VL region (variable light chain region) represented by SEQ ID NO: 14; a gene encoding the FMC63 VH region (variable heavy chain region) represented by SEQ ID NO: 16; and a gene encoding Traptavidin (Tav) represented by SEQ ID NO: 18.

In addition, the vector for manufacturing the antibody-like protein includes a third linker gene represented by the 388th to 432nd nucleotide sequences of SEQ ID NO: 19 between the FMC63 VL gene and the FMC63 VH gene, includes an SfiI gene represented by SEQ ID NO: 21 and a fourth linker gene represented by the 808th to 861st nucleotide sequences of SEQ ID NO: 19 between the FMC63 HL gene and the Tav gene, and includes a His tag gene represented by SEQ ID NO: 20 at the 3' end of the gene encoding Traptavidin (Tav). In addition, one or two additional nucleotides for frame shift may be included between the SfiI gene and the fourth linker gene, and specifically, adenosine (A) and guanine (G) may be sequentially included as additional nucleotides between the SfiI gene and the fourth linker gene.

Specifically, the vector for manufacturing the antibody-like protein of the present invention may include a polynucleotide represented by SEQ ID NO: 19.

In addition, the present invention provides an immune cell therapeutic composition for preventing or treating cancer, the composition comprising: an immune cell comprising a chimeric antigen receptor comprising a tag protein, an HTM region (hinge and transmembrane region), a costimulatory region, and a stimulatory region, and expressing the tag protein on a cell membrane surface; and an antibody-like protein comprising a single chain variable fragment (scFv) and Traptavidin.

The antibody-like protein forms a tetramer due to self-assembly of the Traptavidin, and the Traptavidin forming the tetramer binds to the tag protein expressed on the surface of the immune cell so that the immune cell has a form in which four single chain variable fragments (scFv) are exposed on the surface of the immune cell. Therefore, since the immune cell therapeutic of the present invention has four single chain variable fragments (scFv), binding ability (avidity) to a tumor-specific antigen is enhanced, and the anti-cancer effect is excellent compared with a conventional cell therapeutic.

The cancer may be one or more diseases selected from the group consisting of leukemia, acute lymphocytic leukemia, acute myelogenous leukemia, chronic leukemia, chronic myelogenous (granulocytic) leukemia, chronic lymphocytic leukemia, polycythemia vera, lymphoma, Hodgkin disease, non-Hodgkin disease, multiple myeloma, Waldenstrom macroglobulinemia, heavy chain disease, fibrosarcoma, myxosarcoma, liposarcoma, chondrosarcoma, osteosarcoma, chordoma, angiosarcoma, endothelial sarcoma, lymphangiosarcoma, lymphangioendotheliosarcoma, synovioma, mesothelioma, Ewing tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinoma, cystadenocarcinoma, medullary carcinoma, bronchogenic carcinoma, renal cell carcinoma, liver tumor, bile duct carcinoma, choriocarcinoma, seminoma, embryonal carcinoma, Wilm tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

The pharmaceutical composition of the present invention may be manufactured in a unit dosage form by formulation using a pharmaceutically acceptable carrier according to a method that can be easily carried out by a person having ordinary skill in the technical field to which the present invention pertains, or may be manufactured by being placed in a multi-dose container.

The pharmaceutically acceptable carrier is conventionally used in formulation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. The pharmaceutical composition of the present invention may further include lubricants, wetting agents, sweeteners, flavoring agents, emulsifying agents, suspending agents, preservatives, and the like in addition to the above components.

In the present invention, the content of the additive included in the pharmaceutical composition is not particularly limited, and may be appropriately adjusted within a content range used for conventional formulation.

The pharmaceutical composition may be formulated in an injectable dosage form such as an aqueous solution, a suspension, an emulsion, or the like, or in one or more external preparation forms selected from the group consisting of pills, capsules, granules, tablets, creams, gels, patches, sprays, ointments, plasters, lotions, liniments, pastes, and cataplasms.

The pharmaceutical composition of the present invention may include additional pharmaceutically acceptable carriers and diluents for formulation. The pharmaceutically acceptable carriers and diluents include, but are not limited to, excipients such as starch, sugar, and mannitol, fillers and extenders such as calcium phosphate, cellulose derivatives such as carboxymethylcellulose and hydroxypropylcellulose, binders such as gelatin, alginate, and polyvinylpyrrolidone, lubricants such as talc, calcium stearate, hydrogenated castor oil, and polyethylene glycol, disintegrants such as povidone and crospovidone, and surfactants such as polysorbate, cetyl alcohol, and glycerol. The pharmaceutically acceptable carriers and diluents may be biologically and physiologically compatible with a subject. Examples of the diluent include, but are not limited to, saline, aqueous buffer solutions, solvents, and/or dispersion media.

The pharmaceutical composition of the present invention may be orally administered or parenterally administered (for example, intravenously, subcutaneously, intraperitoneally, or topically applied) according to a desired method. In the case of oral administration, the pharmaceutical composition may be formulated as tablets, troches, lozenges, aqueous suspensions, oily suspensions, prepared powders, granules, emulsions, hard capsules, soft capsules, syrups, elixirs, or the like. In the case of parenteral administration, the pharmaceutical composition may be formulated as injections, suppositories, powders for respiratory inhalation, aerosol agents for spray, ointments, powders for application, oils, creams, or the like.

The dosage of the pharmaceutical composition of the present invention may vary in range depending on a patient's condition and weight, age, sex, health condition, dietary constitution specificity, nature of the preparation, degree of disease, administration time of the composition, administration method, administration period or interval, excretion rate, and drug form, and may be appropriately selected by a person having ordinary skill in the art. For example, the dosage may be in a range of about 0.1 to 10,000 mg/kg, but is not limited thereto, and may be administered once a day to several times a day in divided doses.

The pharmaceutical composition may be orally administered or parenterally administered (for example, intravenously, subcutaneously, intraperitoneally, or topically applied) according to a desired method. A pharmaceutically effective amount and an effective dosage of the pharmaceutical composition of the present invention may vary depending on a formulation method, administration mode, administration time, and/or administration route of the pharmaceutical composition, and a person having ordinary skill in the technical field may easily determine and prescribe a dosage effective for a desired treatment. Administration of the pharmaceutical composition of the present invention may be performed once a day, or may be performed in divided doses several times.

### MODE FOR CARRYING OUT THE INVENTION

Hereinafter, examples will be described in detail in order to help understanding of the present invention. However, the following examples merely illustrate the content of the present invention, and the scope of the present invention is not limited to the following examples. The examples of the present invention are provided to more completely describe the present invention to a person having average knowledge in the art.

### Example 1. Production of Immune Cells Expressing Chimeric Antigen Receptor

### 1-1. Preparation of Plasmid for CAR Expression

The chimeric antigen receptor (hereinafter, CAR) according to the present invention is sequentially composed of a tag protein, a hinge and transmembrane region (hereinafter, HTM), a costimulatory region, and a stimulatory region, and in the present example, as one aspect of the CAR, was produced to sequentially include an Avidin tag protein, CD8 HTM, 4-1BB, and CD3ζ.

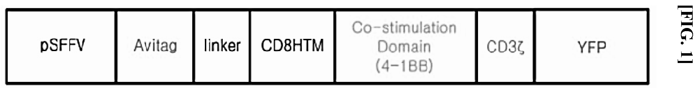

In the present example, a plasmid comprising an inserted DNA encoding the CAR protein was prepared (FIG. 1). The inserted DNA was designed to sequentially include an Avidin gene encoding an Avidin tag protein represented by SEQ ID NO: 2, a CD8 HTM gene encoding CD8 HTM represented by SEQ ID NO: 4, a 4-1BB gene encoding 4-1BB represented by SEQ ID NO: 6, and a CD3ζ gene encoding CD3ζ represented by SEQ ID NO: 8. In the inserted DNA, a first linker gene represented by the 46th to 51st nucleotide sequences of SEQ ID NO: 12 was designed to exist between the Avidin gene and the CD8 HTM gene. In addition, in the present example, in order to easily confirm expression and insertion of the CAR by a fluorescent protein, a gene encoding GFP (green fluorescent protein) represented by SEQ ID NO: 10 was included at the 3' end of the CD3ζ gene, which is a stimulatory region, and a second linker gene represented by the 721st to 735th nucleotide sequences of SEQ ID NO: 12 was designed to exist between the CD3ζ gene and the GFP gene.

Finally, the inserted DNA encoding the CAR prepared in the present example is composed of a nucleotide sequence represented by SEQ ID NO: 12, and the finally manufactured CAR protein was designed to have an amino acid sequence represented by SEQ ID NO: 11. Each gene was cloned into a pHR-SFFV vector (Addgene) by requesting a sequencing service, thereby preparing a pHR-SFFV-CAR-GFP recombinant vector, and the presence of the inserted DNA was confirmed using agarose gel electrophoresis.

### 1-2. Preparation of Lentivirus for CAR Infection

A lentivirus capable of transducing the manufactured pHR-SFFV-CAR-GFP recombinant vector into immune cells was prepared. 293T cells at a concentration of 6 × 10⁶ cell/mL were prepared in a 100π dish, and the pHR-SFFV-CAR-GFP recombinant vector, pMD2G (Addgene, 12259), and psPAX2 (Addgene, 12260) were transfected at 8 µg each. The cells were cultured for 3 to 5 days under conditions of 37°C and 5% CO₂, and a culture solution was recovered and centrifuged at 2,000 rpm for 10 minutes at 4°C to separate a supernatant. The separated supernatant was filtered through a 0.45 µm filter, Lenti X concentrator (Takara, 631231) was added to the filtered supernatant in an amount of 1/4, and the mixture was reacted overnight at 4°C. The solution in which the reaction was completed was centrifuged for 45 minutes under conditions of 1,500 xg at 4°C, and the obtained pellet was resuspended in PBS (phosphate buffer saline), thereby preparing a lentivirus for CAR infection at a concentration of 1 × 10¹³ copies/mL.

### 1-3. Culture of Immune Cells

CD4⁺/CD8⁺ T cells were isolated from human PBMC (Human Peripheral Blood Mononuclear Cells) using EasySep^{™} Human T cell Isolation kit (Stemcell, 17951). The isolated CD4⁺/CD8⁺ T cells were seeded at 1 × 10⁶ cells/mL in ImmunoCult XF T Cell Expansion Medium (Stemcell, 10981) comprising 25 µL of Dynabeads human T activator (Gibco, 11131D) and 50 U/mL of IL-2 (Roche, 11011456001) in a 24-well plate, and cultured for 3 days. The cells were counted, and a medium was added so that the cells became 2 × 10⁵ cells/mL, and the cells were cultured.

### 1-4. Preparation of CAR-T Using Lentivirus

The CD4⁺/CD8⁺ T cells after completion of the culture were dispensed at 1 mL each into a 24-well plate at a concentration of 1 × 10⁶ cells/mL, the prepared lentivirus was added at a concentration of 1 × 10⁶ to 1 × 10⁷ copies/mL, 8 µg of polybrene was added, and centrifugation was performed for 70 minutes under conditions of 900 xg at 32°C to induce binding of the lentivirus and T cells. Thereafter, the cells were cultured overnight under conditions of 37°C and 5% CO₂, and then a new medium was added. After culture for 3 days while changing the medium in the same manner as described above, an expression level of GFP was analyzed by flow cytometry to confirm whether CAR-T (Chimeric Antigen Receptor T cell) in which the CAR protein was transduced into CD4⁺/CD8⁺ T cells was produced. As a control group, wild-type CD4⁺/CD8⁺ T cells (No-infection) not infected with lentivirus were used.

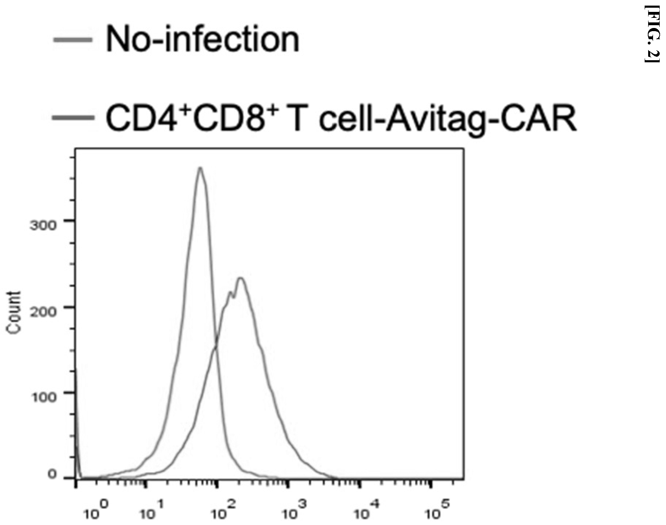

As shown in FIG. 2, unlike the wild-type CD4⁺/CD8⁺ T cells (No-infection), expression of GFP was detected in CAR-T (T cell-Avitag-CAR) transduced with the CAR protein. These results demonstrate that the CAR protein manufactured according to the present invention was transduced into T cells.

### 1-5. Biotin Ligation

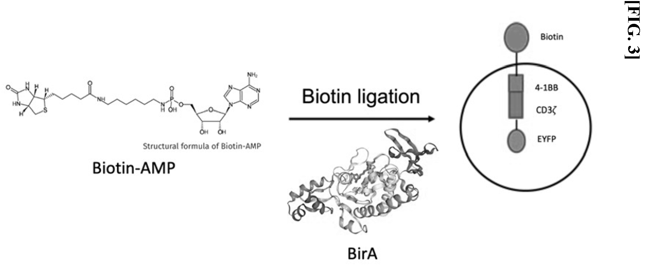

A biotin-tagged CAR-expressing immune cell was produced by ligating a biotin tag to an Avidin tag protein of T cells (CAR-T) transduced with the CAR protein manufactured according to the present invention. Biotin binding was performed using biotin-AMP (biotin-adenosine mono phosphate) and BirA enzyme (FIG. 3). The CAR-T was seeded in 500 µL of PBS (phosphate buffer saline) at a concentration of 1 × 10⁶ cells, 10 µM AMP-biotin (Jena bioscience, NU-894-BIO-S) and 0.3 µM BirA (Abcam, ab135015) were added thereto, and the cells were cultured at 37°C for 1 hour. After the cultured cells were stained with Cyanine5-labeled Traptavidin, whether biotin-tagged CAR-T was produced was confirmed by confocal microscopy.

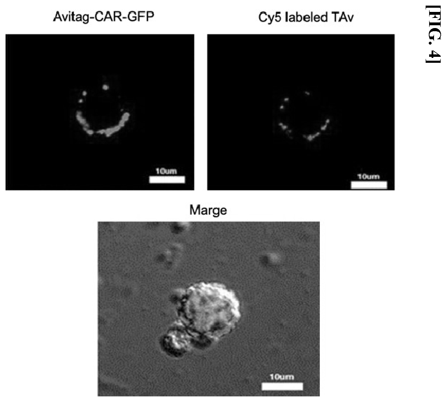

As shown in FIG. 4, fluorescence of GFP (green fluorescent protein) bound to the terminus of the chimeric antigen receptor (CAR) was confirmed on the surface of T cells (CAR-T) transduced with the CAR protein, and fluorescence of Cyanine5-labeled Traptavidin (Cy5 labeled TAv) was detected at the same portion. These results demonstrate that Traptavidin stably binds to the biotin protein exposed on the surface of T cells (CAR-T) transduced with the CAR protein.

### Example 2. Production of Traptavidin Scaffold Antibody-Like Protein

### 2-1. Preparation of FMC63-Tav Recombinant Vector

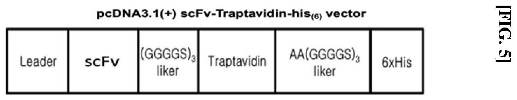

The Traptavidin scaffold antibody-like protein according to the present invention is a protein (scFv-Tav protein) sequentially composed of a single chain variable fragment (hereinafter, scFv) recognizing a cell surface molecule of a specific cancer cell and Traptavidin (hereinafter, Tav), and in the present example, as one aspect of the scFv-Tav protein, was produced to include an scFv of FMC63 and Traptavidin (FIG. 5).

In the present example, a recombinant vector comprising genes encoding respective components was prepared in order to manufacture the scFv-Tav protein, and was designed to sequentially include a leader sequence represented by SEQ ID NO: 22, a gene encoding the FMC63 VL region (variable light chain region) represented by SEQ ID NO: 14, a gene encoding the FMC63 VH region (variable heavy chain region) represented by SEQ ID NO: 16, and a gene encoding Traptavidin (Tav) represented by SEQ ID NO: 18, between an EcoRI enzyme cleavage site (gaattc) and an XhoI enzyme cleavage site (ctcgag). A third linker gene represented by the 388th to 432nd nucleotide sequences of SEQ ID NO: 19 was designed to exist between the FMC63 VL gene and the FMC63 VH gene. Between the FMC63 HL gene and the Tav gene, an SfiI gene represented by SEQ ID NO: 21 and a fourth linker gene represented by the 808th to 861st nucleotide sequences of SEQ ID NO: 19 were designed to exist. One or two additional nucleotides for frame shift may exist between the SfiI gene and the fourth linker gene, and specifically, in the present example, adenosine (A) and guanine (G) were designed to exist as additional nucleotides between the SfiI gene and the fourth linker gene. In addition, in the present example, in order to easily isolate the scFv-Tav protein, a His tag gene represented by SEQ ID NO: 20 was designed to exist at the 3' end of the gene encoding Traptavidin (Tav).

Finally, the gene encoding the scFv-Tav protein prepared in the present example is composed of a nucleotide sequence represented by SEQ ID NO: 19, and each gene was inserted into a pcDNA3.1 vector by requesting a sequencing service, thereby preparing a pcDNA3.1-FMC63-Tav recombinant vector, and the presence of the inserted DNA was confirmed using agarose gel electrophoresis.

### 2-2. Production of scFv-Tav Scaffold Antibody-Like Protein

The prepared recombinant vector was transfected into expression cells to manufacture an FMC63-Tav protein. ExpiCHO cells (Gibco, A29133) in which a vector expression system was established were prepared in a 125 mL flask at 25 mL and 6 × 10⁶ cell/mL. Transfection was performed with the pcDNA3.1-FMC63-Tav recombinant vector according to instructions of the ExpiCHO manufacturer. After culture for 7 to 9 days, a culture solution was obtained. The obtained culture solution was centrifuged for 10 minutes under conditions of 2,900 xg at 4°C to obtain a supernatant. After the obtained supernatant was filtered through a 0.22 µm filter (Sartorius, 17573), the FMC63-Tav protein was purified using FPLC (Fast protein liquid chromatography). Whether the protein was purified was confirmed through SDS-PAGE. The purified FMC63-Tav protein was concentrated using Amicon (Merck, UFC805024), and then suspended in PBS and stored frozen.

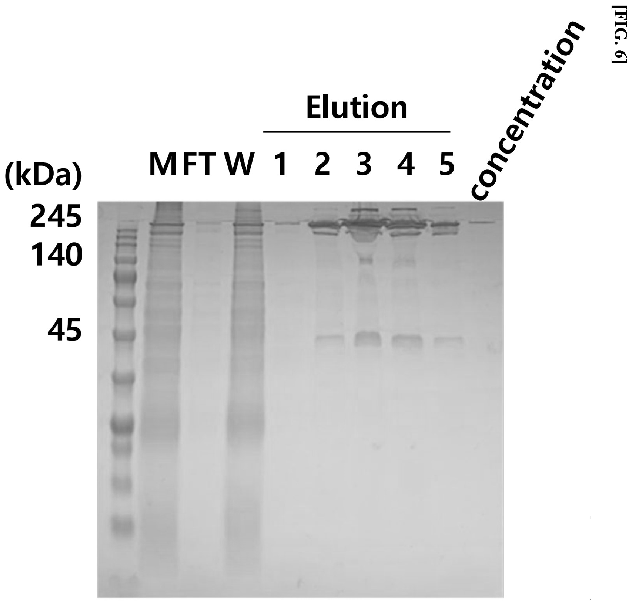

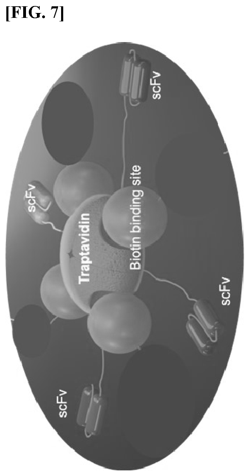

As shown in FIG. 6, a band having a size of 42 kDa corresponding to a single FMC63-Tav (mono-FMC63-Traptavidin) protein was detected in an FPLC elution sample. In addition, a band having a size of 168 kDa, which is a band corresponding to a t-FMC63-Tav protein (FIG. 7) in a tetramer form formed by self-assembly of Traptavidin, was detected.

In order to confirm the configuration of the manufactured FMC63-Tav protein, a biotin binding site in the FMC63-Tav protein was confirmed through Western blot using biotin-HRP (Biotin Horseradish peroxidase). A Traptavidin protein was used as a control group. In addition, the presence of FMC63 was confirmed through Western blot using an anti-FMC63 antibody.

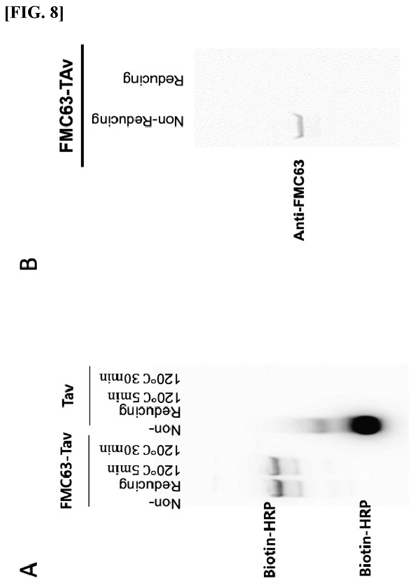

As shown in FIG. 8, it was confirmed that biotin-HRP bound to the manufactured FMC63-Tav protein, and biotin-HRP was also shown to bind to the Traptavidin (Tav) protein. These results demonstrate that, in the manufactured FMC63-Tav protein, the biotin binding site is the Traptavidin (Tav) protein. In addition, as a result of Western blot using an anti-FMC63 antibody, it was confirmed that the anti-FMC63 antibody bound to the manufactured FMC63-Tav protein, thereby demonstrating that FMC63 is present in the manufactured FMC63-Tav protein.

Traptavidin (Tav) is a material having strong resistance to heat, and is not completely denatured even at a temperature of 100°C. As shown in FIG. 8, even under conditions of 120°C for 5 minutes, the band of FMC63-Tav maintains the form of a tetramer, and this demonstrates that denaturation occurs from the linker connecting scFv and Tav. Tav is completely denatured under heating conditions of about 30 minutes, which demonstrates that Tav can be used as an antibody scaffold having very high thermal stability.

### Example 3. Evaluation of Efficacy of Tetramer Universal CAR-Immune Cell Therapeutic

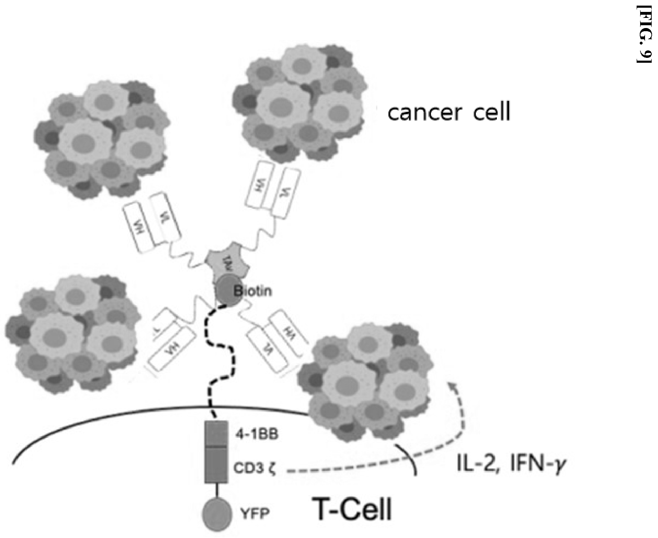

An immune cell therapeutic comprising tetramer based universal CAR-T having four scFv regions was constructed by binding the biotin-tagged CAR-expressing immune cell and the scFv-Tav scaffold antibody-like protein (FIG. 9). Specifically, the FMC63-Tav protein prepared in Example 2 was bound to the biotin-tagged CAR-T prepared in Example 1. After the biotin-tagged CAR-T was washed three times with easySep buffer (Stemcell, 20144), the biotin-tagged CAR-T at a concentration of 1 × 10⁶ and the FMC63-Tav protein at 0.2 µg/µL were added to 500 µL of PBS and reacted at 37°C for 1 hour, thereby finally constructing FMC63-Tav-Bio-CAR-T cells. In order to evaluate anti-cancer activity of the manufactured FMC63-Tav-Bio-CAR-T cells, change in cytokine level and cytotoxicity against cancer cells were evaluated. In order to comparatively evaluate anti-cancer activity, Kymriah, which is a commercially used anti-cancer agent, and activated wild-type T cells (Untransduction) into which CAR was not introduced were used as comparative controls.

### 3-1. Evaluation of Change in Cytokine Level

The manufactured tetramer based universal CAR-T (FMC63-Tav-Bio-CAR-T cells) was applied as effector cells, and changes in the levels of IFN-γ and IL-2, which are cytokines essential for immune anti-cancer action against target cells, CD19⁺ Raji cells, which are a lymphoma cell line, were measured. In a 96-well plate, CD19⁺ Raji cells, which are target cells, were seeded at a concentration of 1 × 10⁴ cell/well in each well. FMC63-Tav-Bio-CAR-T cells, which are effector cells, were seeded in each well so that a ratio (E:T ratio) of the effector cells to the target cells became 0:1, 1:1, 2:1, 5:1, or 10:1, and co-cultured for 48 hours. In the culture solution after completion of culture, the levels of IFN-γ and IL-2 were measured using ELISA (BD, 555142 and 555190).

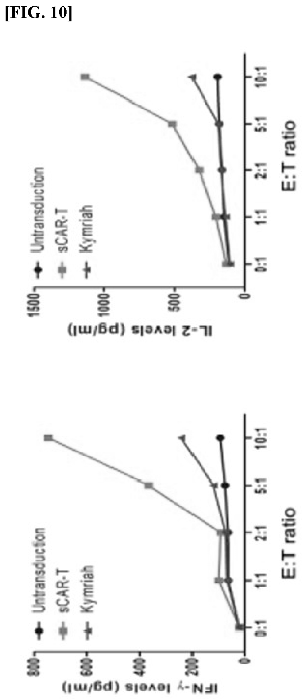

As shown in FIG. 10, it was shown that, when FMC63-Tav-Bio-CAR-T cells were used as effector cells, the levels of cytokines IFN-γ and IL-2 were significantly increased compared with those of Kymriah and wild-type T cells (Untransduction). These results demonstrate that the tetramer based universal CAR immune cell therapeutic manufactured according to the present invention has excellent immune anti-cancer response compared with a conventional commercial anti-cancer agent and wild-type T cells, thereby exhibiting excellent anti-cancer activity.

### 3-2. Cytotoxicity Evaluation

The cytotoxicity effect against target cells, CD19⁺ Raji cells, which are a lymphoma cell line, was evaluated by applying the manufactured FMC63-Tav-Bio-CAR-T cells as effector cells. Raji cells were transduced using lentivirus so that nanoLuc (nano luciferase) was expressed. Thereafter, in a 96-well plate, CD19⁺ Raji cells, which are target cells, were seeded at a concentration of 1 × 10⁴ cell/well in each well. FMC63-Tav-Bio-CAR-T cells, which are effector cells, were seeded in each well so that a ratio (E:T ratio) of the effector cells to the target cells became 0:1, 1:1, 2:1, 5:1, or 10:1, and co-cultured for 48 hours. The cells after completion of culture were lysed using Passive lysis buffer (Promega, E1941), and then a luminescence level was measured using nanoluc substrate (Promega, N1150) to measure a reduction level of CD19⁺ Raji cells. The presence of CD19⁺ Raji cells was calculated as a relative % based on 100% in a case where effector cells were not added.

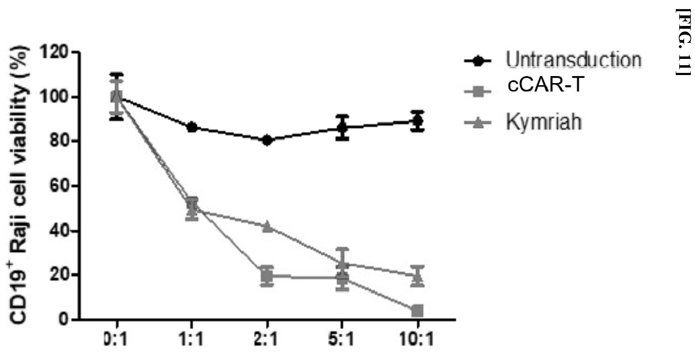

As shown in FIG. 11, it was shown that, when FMC63-Tav-Bio-CAR-T cells were used as effector cells, CD19⁺ Raji cells, which are a lymphoma cell line, rapidly decreased compared with Kymriah and wild-type T cells (Untransduction). These results demonstrate that the tetramer based universal CAR immune cell therapeutic manufactured according to the present invention has excellent immune anti-cancer response compared with a conventional commercial anti-cancer agent and wild-type T cells, thereby exhibiting excellent anti-cancer activity.

As described above, specific portions of the content of the present invention have been described in detail. It will be apparent to those having ordinary skill in the art that such specific description is merely a preferred embodiment and that the scope of the present invention is not limited thereby. That is, the substantial scope of the present invention is defined by the appended claims and equivalents thereof.

## Claims

1. A chimeric antigen receptor comprising a tag protein, an HTM region (hinge and transmembrane region), a costimulatory region, and a stimulatory region.

2. The chimeric antigen receptor according to claim 1,
wherein the tag protein is an Avidin or a Biotin tag protein.

3. The chimeric antigen receptor according to claim 1,
wherein the HTM region (hinge and transmembrane region) is CD8 HTM represented by SEQ ID NO: 3.

4. The chimeric antigen receptor according to claim 1,
wherein the costimulatory region is one or more proteins selected from the group consisting of 4-1BB, CD2, CD7, CD27, CD28, CD30, CD40, CD83, CD258, NKG2C, NKG2D, B7-H3, OX40, ICAM-1, LFA-1, and ICOS, or active regions thereof.

5. The chimeric antigen receptor according to claim 1,
wherein the costimulatory region is 4-1BB represented by SEQ ID NO: 5.

6. The chimeric antigen receptor according to claim 1,
wherein the stimulatory region is one or more proteins selected from the group consisting of CD3ζ, CD3ε, CD3γ, CD3δ, TCRα, TCRβ, TCRδ, TCRγ, CD79a, CD79b, DAP10, and DAP12, or active regions thereof.

7. The chimeric antigen receptor according to claim 1,
wherein the stimulatory region is CD3ζ represented by SEQ ID NO: 7.

8. The chimeric antigen receptor according to claim 1,
wherein the chimeric antigen receptor is a polypeptide represented by SEQ ID NO: 11.

9. A polynucleotide encoding the chimeric antigen receptor according to any one of claims 1 to 8.

10. A vector for manufacturing a chimeric antigen receptor comprising the polynucleotide according to claim 9.

11. The vector for manufacturing the chimeric antigen receptor according to claim 10,
wherein the vector for manufacturing the chimeric antigen receptor comprises:
an Avidin gene encoding an Avidin tag protein represented by SEQ ID NO: 2;
a CD8 HTM gene encoding CD8 HTM represented by SEQ ID NO: 4;
a 4-1BB gene encoding 4-1BB represented by SEQ ID NO: 6; and
a CD3ζ gene encoding CD3ζ represented by SEQ ID NO: 8.

12. The vector for manufacturing the chimeric antigen receptor according to claim 11,
wherein the vector for manufacturing the chimeric antigen receptor comprises a first linker gene represented by nucleotide positions 46 to 51 of SEQ ID NO: 12 between the Avidin gene and the CD8 HTM gene.

13. The vector for manufacturing the chimeric antigen receptor according to claim 10,
wherein the vector for manufacturing the chimeric antigen receptor comprises a polynucleotide represented by SEQ ID NO: 12.

14. The vector for manufacturing the chimeric antigen receptor according to claim 10,
wherein the vector for manufacturing the chimeric antigen receptor is a lentiviral vector, a retroviral vector, a DNA vector, a plasmid, an RNA vector, an adenoviral vector, or an adeno-associated viral vector.

15. An immune cell comprising a chimeric antigen receptor comprising a tag protein, an HTM region (hinge and transmembrane region), a costimulatory region, and a stimulatory region, and expressing the tag protein on a cell membrane surface.

16. The immune cell according to claim 15,
wherein the tag protein is an Avidin or a Biotin tag protein.

17. The immune cell according to claim 15,
wherein the HTM region (hinge and transmembrane region) is CD8 HTM represented by SEQ ID NO: 3, the costimulatory region is 4-1BB represented by SEQ ID NO: 5, and the stimulatory region is CD3ζ represented by SEQ ID NO: 7.

18. The immune cell according to claim 15,
wherein the immune cell is a T cell, an NK cell, or a B cell.

19. A method for manufacturing a CAR-expressing immune cell, the method comprising transducing an immune cell with a vector comprising a polynucleotide encoding a chimeric antigen receptor comprising a tag protein, an HTM region (hinge and transmembrane region), a costimulatory region, and a stimulatory region.

20. The method for manufacturing the CAR-expressing immune cell according to claim 19,
wherein when the tag protein is an Avidin, the method further comprises, after the transducing step, a Biotin ligation step by reacting with biotin AMP (biotin-adenosine mono phosphate).

21. An antibody-like protein comprising a single chain variable fragment (scFv) and Traptavidin.

22. The antibody-like protein according to claim 21,
wherein the single chain variable fragment (scFv) binds to a tumor-specific antigen.

23. The antibody-like protein according to claim 22,
wherein the tumor-specific antigen is CD19, CD20, GD2, HER-2, CD30, EGFR, FAP, CD33, CD123, PD-L1, IGF1R, CSPG4, or B7-H4.

24. The antibody-like protein according to claim 21,
wherein the single chain variable fragment (scFv) comprises an FMC63 VL region (variable light chain region), which is a polypeptide represented by SEQ ID NO: 13, and an FMC63 VH region (variable heavy chain region), which is a polypeptide represented by SEQ ID NO: 15.

25. The antibody-like protein according to claim 21,
wherein the Traptavidin is a polypeptide represented by SEQ ID NO: 17.

26. A polynucleotide encoding the antibody-like protein according to any one of claims 21 to 25.

27. A vector for manufacturing an antibody-like protein comprising the polynucleotide according to claim 26.

28. The vector for manufacturing the antibody-like protein according to claim 27,
wherein the vector for manufacturing the antibody-like protein comprises:
a leader sequence represented by SEQ ID NO: 22;
a gene encoding an FMC63 VL region (variable light chain region) represented by SEQ ID NO: 14;
a gene encoding an FMC63 VH region (variable heavy chain region) represented by SEQ ID NO: 16; and
a gene encoding Traptavidin (Tav) represented by SEQ ID NO: 18.

29. The vector for manufacturing the antibody-like protein according to claim 28,
wherein the vector for manufacturing the antibody-like protein comprises:
a third linker gene represented by nucleotide positions 388 to 432 of SEQ ID NO: 19 between the FMC63 VL gene and the FMC63 VH gene,
an SfiI gene represented by SEQ ID NO: 21 and a fourth linker gene represented by nucleotide positions 808 to 861 of SEQ ID NO: 19 between the FMC63 HL gene and the Tav gene, and
a His tag gene represented by SEQ ID NO: 20 at the 3' end of the gene encoding Traptavidin (Tav).

30. The vector for manufacturing the antibody-like protein according to claim 29,
wherein one or two additional nucleotides for frame shift are included between the SfiI gene and the fourth linker gene.

31. The vector for manufacturing the antibody-like protein according to claim 27,
wherein the vector for manufacturing the antibody-like protein comprises a polynucleotide represented by SEQ ID NO: 19.

32. An immune cell therapeutic composition for preventing or treating cancer, the composition comprising:
an immune cell comprising a chimeric antigen receptor comprising a tag protein, an HTM region (hinge and transmembrane region), a costimulatory region, and a stimulatory region, and expressing the tag protein on a cell membrane surface; and
an antibody-like protein comprising a single chain variable fragment (scFv) and Traptavidin.

33. The immune cell therapeutic composition for preventing or treating cancer according to claim 32,
wherein the antibody-like protein forms a tetramer due to self-assembly of the Traptavidin, and the Traptavidin that forms the tetramer binds to the tag protein expressed on the surface of the immune cell.

34. The immune cell therapeutic composition for preventing or treating cancer according to claim 32,
wherein the tag protein is a Biotin tag protein, the HTM region (hinge and transmembrane region) is CD8 HTM represented by SEQ ID NO: 3, the costimulatory region is 4-1BB represented by SEQ ID NO: 5, and the stimulatory region is CD3ζ represented by SEQ ID NO: 7.

35. The immune cell therapeutic composition for preventing or treating cancer according to claim 32,
wherein the immune cell is a T cell, an NK cell, or a B cell.

36. The immune cell therapeutic composition for preventing or treating cancer according to claim 32,
wherein the single chain variable fragment (scFv) binds to a tumor-specific antigen.

37. The immune cell therapeutic composition for preventing or treating cancer according to claim 36, wherein the tumor-specific antigen is CD19, CD20, GD2, HER-2, CD30, EGFR, FAP, CD33, CD123, PD-L1, IGF1R, CSPG4, or B7-H4.

38. The immune cell therapeutic composition for preventing or treating cancer according to claim 32,
wherein the single chain variable fragment (scFv) comprises an FMC63 VL region (variable light chain region), which is a polypeptide represented by SEQ ID NO: 13, and an FMC63 VH region (variable heavy chain region), which is a polypeptide represented by SEQ ID NO: 15.

39. The immune cell therapeutic composition for preventing or treating cancer according to claim 32,
wherein the cancer is one or more diseases selected from the group consisting of leukemia, acute lymphocytic leukemia, acute myelogenous leukemia, chronic leukemia, chronic myelogenous (granulocytic) leukemia, chronic lymphocytic leukemia, polycythemia vera, lymphoma, Hodgkin disease, non-Hodgkin disease, multiple myeloma, Waldenstrom macroglobulinemia, heavy chain disease, fibrosarcoma, myxosarcoma, liposarcoma, chondrosarcoma, osteosarcoma, chordoma, angiosarcoma, endothelial sarcoma, lymphangiosarcoma, lymphangioendotheliosarcoma, synovioma, mesothelioma, Ewing tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinoma, cystadenocarcinoma, medullary carcinoma, bronchogenic carcinoma, renal cell carcinoma, liver tumor, bile duct carcinoma, choriocarcinoma, seminoma, embryonal carcinoma, Wilm tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.
